# EUROPEAN PATENT APPLICATION

(11) **EP 1 958 636 A1**
(43) Date of publication of application: **20.08.2008**
(21) Application number: 06769371.3
(22) Date of filing: 23.06.2006
(51) Int. Cl.: A61K 31/64, A61K 31/4439, A61P 3/10

(54) **PHARMACEUTICAL COMPOSITIONS CONTAINING COMBINED ANTIDIABETIC SUBSTANCES FOR USE IN TYPE 2 DIABETES MELLITUS**

(30) Priority: 06.12.2005 MX PA05013220
(71) Applicant: ESPINOZA ABDALA, Leopoldo de Jesús, Jalisco Mexico (MX)
(72) Inventor: GARCÍA ARMENTA, María Elena, C.P. 45020 Guadalajara, Jalisco (MX); ÁLVAREZ OCHOA, Víctor Guillermo, C.P. 45222 Zapopan, Jalisco (MX); SANTOS MURILLO, Josefina, C.P. 44600 Zapopan, Jalisco (MX)
(74) Representative: Carvajal y Urquijo, Isabel
(86) International application number: PCT/MX2006/000060
(87) International publication number: WO 2007/067027

(57) **Abstract**

The invention relates to a pharmaceutical composition which combines antidiabetic substances such as a sulphonylurea such as glimepiride and a thiazolidinedione such as pioglitazone and which is intended for pharmacological use in the treatment of type 2 diabetes mellitus. The aforementioned composition produces a combined product having improved properties, as well as producing a synergic effect. In summary, the invention comprises a synergic pharmaceutical composition containing a synergic combination of a sulphonylurea and a thiazolidinedione for the treatment of type 2 diabetes mellitus, in which the sulphonylurea is glimepiride and/or any of the salts thereof and the thiazolidinedione is pioglitazone and/or any of the salts thereof.

## Description

### FIELD OF THE INVENTION

The present invention describes a pharmaceutical composition comprising a combination of antidiabetic substances, as a sulphonyl urea as Glimepiride, and a thiazolidinedione as Pioglitazone, for the pharmacological use of the composition in treatment of Type 2 Diabetes Mellitus; the composition produces a combination product with improved properties and produces a synergistic effect.

### BACKGROUND OF THE INVENTION

The diabetes mellitus (DM) is an important problem of public health in Mexico. The tendency to increase becomes worse in 1998. In the last five years it has ended up occupying the first cause of death with 11% of total deceases in both female and male, and 14% of deaths are women. According to data of Diabetes statistics in Mexico, the causes of death are complications of DM:

| **COMPLICATIONS OF DM** | **CIE*** | |
|---|---|---|
| | **1997** | **2001** |
| | **%** | **%** |
| Coma | 3.9 | 4.5 |
| Cetoacidosis | 5.2 | 3.2 |
| Renal complications | 30.0 | 30.4 |
| Ophthalmic complications | 0.0 | 0.0 |
| Neurological complications | 0.3 | 0.2 |
| Comp. of peripheral circulation | 1.4 | 1.3 |
| Other specified complications | 20.7 | 17.6 |
| Multiple complications | 21.5 | 24.4 |
| Unspecific complications | 2.8 | 3.6 |
| Without mention of complications | 14.1 | 14.9 |

- ICD = International Classification of Diseases.
- Source CEMECE, 2002.

Is important to note that renal complications of DM are the major cause of death in these patients, for this reason the therapy of DM should be focused to glucose control and prevention of complications.

The chronic complications occur after having had high glucose in blood during lengthy (years) periods and they are:
Diabetic retinopathy.
Diabetic nephropathy.
Diabetic neuropathy.
Cardiovascular complications.

As mentioned above the fundamental aspects in treatment are: diet and weight control, bodily activity, and therapy with medications.

There are different types of medications used in therapy, however, it has been observed that the combined therapy of medications are mostly effective in this pathology, compared to monotherapy used, since with this last one, it has not been found an effective control of glycemic index.

As discussed, there are several within the mostly used medications, however, it is of our interest to revise sulphonyl ureas and thiazolidinediones.

**SULPHONYL UREAS**: All of the drugs of this group are derived from the sulfamides, in which the structure of sulphonyl urea constitutes the essential group of the hypoglycemiant action. Sulphonyl ureas are known by their hypoglycemiant activity, they are considered the choosing drugs in patients with type 2 DM of relatively recent (< 5 years) beginning, that have an endogenous production of insulin, without obesity and that do not respond appropriately to dietary treatment. The sulphonyl ureas stimulate beta cells of pancreas so as to release more insulin. Sulphonyl ureas are used since the decade of 1950. The chlorpropamide is the only sulphonyl urea of first generation that is still used.

The sulphonyl ureas of second generation are: Glipizide, Glyburide, Glimepiride. These medications are administered one to twice a day before food. All of the sulphonyl ureas produce similar effects on glucose level in blood, but they differ in secondary effects, frequency with which they are administered, and interactions with other medications.

Glimepiride decreases the glucose concentration in blood, by stimulating the release of insulin from the pancreatic beta cells, both in healthy subjects and in patients with type 2 diabetes Mellitus. This effect is mainly due to the pancreatic beta cells response, which increases by the physiologic stimulus of glucose. Glimepiride chemically is 1-[[p-[2-(3-ethyl-4-methyl-2-oxo-3-pyrrolin-1-carboxamido)ethyl]Phenyl]sulphonyl]-3-(trans-4-methylcyclohexyl)urea. It has a molecular weight of 490.62; it generally is a white crystalline powder whose molecular structure is the following:

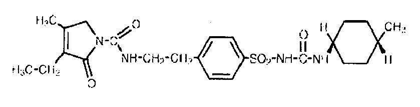

**THIAZOLIDINEDIONES OR GLITAZONES:** They are a new group of hypoglycemiants that are characterized by sensitize or increase the action of insulin without increase its secretion; hence they are useful in situations in which peripheral resistance to insulin is developed. The thiazolidinediones act by improving the sensibility to insulin in patients that have developed resistance to insulin, diminishing the levels of glycemia, insulin and HbA1c, without ending up producing hypoglycemia. They are indicated in patients with type 2 DM with resistance to insulin or in patients that are not well controlled with other forms of therapy. They can be used in monotherapy or in association with insulin and other hypoglycemiants.

Pioglitazone integrates this group; it diminishes the resistance to insulin in periphery and liver, and gives as a result an increase of disposition of insulin-dependent glucose, as well as a decrease in the expense of hepatic glucose. It helps insulin to work well in muscle and fat, and also it reduces the production of glucose in liver. Chemically it is the [(±)-5-[[4-[2-(5-ethyl-2-pyridinyl)ethoxi]phenyl]methyl]-2,4-]thiazolidine-dione, and is a white crystalline powder whose molecular structure is the following:

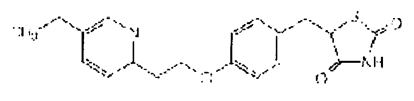

### Physiopathology of type 2 Diabetes:

Most of longitudinal studies have shown clearly that resistance to insulin is the major factor of risk for development of diabetes type 2. In a prospective study of Pima Indians, Lillioja et al evaluated the relative role of obesity, resistance to insulin and dysfunction of β cells in the development of type 2 diabetes mellitus in subjects with normal tolerance to glucose or insufficient tolerance to glucose. They concluded that resistance to insulin was a major factor of risk for diabetes development, with the secretion of insulin being an additional, but weak, factor of risk. The most important argument that supports the resistance to insulin as the primary major genetic factor that lead to diabetes is the observations where it appears that this problem precedes to insufficient function of β cells.

To summarize, the resistance to insulin seems to be explained mostly by the presence of obesity. In fact, the weight reduction is associated with normalization of sensibility to insulin. On the other hand, the disfunction that is present years before of appearance of glucose intolerance and any intervention is available to correct this abnormality. This could support the concept that the insufficiency of β cells is the primary defect that leads to development of diabetes. Resistance to insulin acquired by obesity and the decrease of physical activity accelerate the progression to diabetes. This could explain the epidemic burst of diabetes in a world with fatty foods and more sedentary lifestyle. Therefore, the initial treatments are focused to weight reduction, bodily activity and diet as well as pharmacological treatment.

A coarse majority of patients that use combinations of medications joined to diets and bodily activity have reported adverse events; among those more common are the severe hypoglycemia that includes fine tremor, sweating, nausea and vomit that can end up being serious; this is due to drug interactions, because some combinations enhance the hypoglycemiant effect. For this reason we carried out tests with our combination with the aim of evaluating the synergy of both compounds, efficacy on glycemic indexes; as well as tolerance, evaluating the adverse effects, using fewer doses of ingredients.

### DETAILED DESCRIPTION OF THE INVENTION

According to the present invention, we realized a pharmacological composition that comprise a combination of a well-known sulphonyl urea as Glimepiride and a well-known thiazolidinedione as Pioglitazone, whose combination produces a synergistic effect on indexes of glycosylated hemoglobin, with fewer quantity of ingredients, is tolerated well and with fewer adverse effects in patients with type 2 diabetes Mellitus.

### EXAMPLE 1:

We carry out a comparative study to evaluate the efficacy and tolerance of a combination of sulphonyl urea with a thiazolidinedione as Glimepiride / Pioglitazone, compared to administration of a thiazolidinedione, Pioglitazone alone, in treatment of patients with type 2 diabetes Mellitus. The study last 16 weeks, was double blind, and included 20 patients with diagnosis of type 2 diabetes Mellitus, with glycosylated hemoglobin > or = 8.0%, with elevation of triglyceride levels; the patients were randomized once to receive the Group 1 (n=10), the combination a day, the Group 2 (n=10) was assigned to receive Pioglitazone alone twice a day.

### Results:

The patients that received the combination of Glimepiride / Pioglitazone had a significant decrease of the basal (P = < 0.05) levels in glycosylated Hemoglobin as compared to the group of Pioglitazone alone. The glycosylated Hemoglobin decreased to 0.9% (Confidence Interval 95%): 0.06% to 1.2% with the combination. The group that received the combination had a reduction percentage of triglyceride levels of (26%, IC 95%, 16% to 36%) and an increase in high-density lipid levels (13%, IC, 8% to 18%) as compared to the group that received Pioglitazone alone. In the group of combination there was a small but significant increase of the low-density lipoprotein levels. The group 1 did not report adverse events, the group 2 reported 3 patients with hypoglycemia that was not severe and that ameliorate with administration of honey. There was no necessity to suspend them from study.

### Conclusions:

In patients with type 2 diabetes Mellitus, the combination of Glimepiride / Pioglitazone significantly improves the glycosylated Hemoglobin and plasmatic levels of glucose with beneficial effects on the levels of cholesterol and triglycerides, without adverse events.

### EXAMPLE 2:

We carry out a comparative study to evaluate the efficacy and tolerance of a combination of sulphonyl urea with a thiazolidinedione as Glimepiride / Pioglitazone, as compared to the administration of a sulphonyl urea, Glimepiride alone, in the treatment of patients with type 2 diabetes Mellitus. The study last 16 weeks, was double blind, and included 20 patients with diagnosis of type 2 diabetes Mellitus, with glycosylated hemoglobin > or = 8.0%, with elevation of triglyceride levels; the patients were randomized once to receive the Group 1 (n=10), the combination a day, the Group 2 (n=10) was assigned to receive Glimepiride alone twice a day.

### Results:

The patients that received the combination of Glimepiride / Pioglitazone had a significant decrease of basal (P = < 0.05) levels in glycosylated Hemoglobin as compared to the group of Glimepiride alone. The glycosylated Hemoglobin decreased to 0.8% (Confidence Interval 95%): 0.08% to 1.4% with the combination. The group that received the combination had a reduction percentage of triglyceride levels of (25%, 95% IC, 15% to 34%) and an increase in the high-density lipid levels (13%, IC, 8% to 18%) as compared to the group that received Glimepiride alone. In the group of combination there was not increase of the low-density lipoprotein levels. The group 1 did not report adverse events, the group 2 reported 5 patients with adverse events of hypoglycemia, which suspended the treatment and were reallocated to receive the combination, which was tolerated well without reporting adverse events.

### Conclusions:

In patients with type 2 diabetes Mellitus the combination of Glimepiride / Pioglitazone significantly improves the glycosylated Hemoglobin and the plasmatic levels of glucose with beneficial effects on the levels of cholesterol and triglycerides and without presenting adverse events.

### NOVELTY OF THE INVENTION

Having described the present invention, is considered as novelty and, therefore, is related as property that contained in the following claims:

## Claims

1. A synergistic pharmaceutical composition comprising a synergistic combination of a sulphonyl urea and a thiazolidinedione for the treatment of Type 2 Diabetes Mellitus.

2. The pharmaceutical composition in accordance with claim 1 **characterized in that** it comprises the synergistic combination, wherein the sulphonyl urea is Glimepiride and/or any of their salts and Thiazolidinedione is Pioglitazone and/or any of their salts.

3. A pharmaceutical composition in accordance with claim 2 **characterized** further in that its combination of ingredients improves significantly the plasmatic levels of glucose with beneficial effects on the levels of cholesterol and triglycerides, without adverse events.

4. A pharmaceutical composition in accordance with claim 3 **characterized in that** Pioglitazone is found as Pioglitazone hydrochloride.

5. A pharmaceutical composition in accordance with claim 2 **characterized in that** Pioglitazone is present in a range of 5 mg to 35 mg per dosage unit.

6. A pharmaceutical composition in accordance with claim 5 **characterized in that** Pioglitazone is present in a concentration of 15 mg per dosage unit.

7. A pharmaceutical composition in accordance with claim 7 **characterized in that** Pioglitazone is present in a concentration of 30 mg per dosage unit.

8. A pharmaceutical composition in accordance with claim 2 **characterized in that** Glimepiride is present in a concentration fewer than 6 mg of Glimepiride per dosage unit.

9. A pharmaceutical composition in accordance with claim 10 wherein Glimepiride is present in a concentration of 2 mg per dosage unit.

10. A pharmaceutical composition in accordance with claim 10 wherein Glimepiride is present in a concentration of 4 mg per dosage unit.

11. A pharmaceutical composition in accordance with claim 2 wherein Pioglitazone is present in a percentage of 2.7% to 19%, preferably 8% to 16%.

12. A pharmaceutical composition in accordance with claim 13 wherein the percentage of Pioglitazone is 16%.

13. A pharmaceutical composition in accordance with claim 13 wherein the percentage of Pioglitazone is 8%.

14. A pharmaceutical composition in accordance with claim 2 wherein Glimepiride is present in a percentage of 0.5% to 3%, preferably 1% to 2.5%.

15. A pharmaceutical composition in accordance with claim 16 wherein the percentage of Glimepiride is 1.11%.

16. A pharmaceutical composition in accordance with claim 16 wherein the percentage of Glimepiride is 2.22%.

17. The pharmaceutical compositions in accordance with all of the previous claims **characterized in that** of the formulation are excluded inhibitory substances of HMG-CoA reductase such as Pravastatin, Cerivastatin, Nivastatin, Vibastatin, Lovastatin, Simustatin, Fluvastatin, Rivastatin and Atorvastatin.

18. The pharmaceutical compositions in accordance with all of the previous claims **characterized in that** being coating tablets, Pioglitazone is not part of coating of the same.

19. The pharmaceutical compositions in accordance with all of the previous claims **characterized in that** Biguanides such as Metformin, Buformin or Fenformin are excluded from the formulation.

20. The pharmaceutical compositions in accordance with all of the previous claims **characterized in that** substances such as Fenofibrate, Gemfibrozil, Clofibrate, Clorpropamide, Glicazide, Acarbose, miglitol, Insulin and Implitapid are excluded from the formulation.

21. A pharmaceutical composition in accordance with all of the previous claims **characterized in that** the mixture with any hypolipidemic agent and/or agent for the treatment of the obesity, are excluded from the same.

22. The use of compositions in accordance with claim 2, for the treatment of type 2 diabetes mellitus.
